# EUROPÄISCHE PATENTSCHRIFT

(11) **EP 3 194 065 B1**
(45) Veröffentlichungstag und Bekanntmachung des Hinweises auf die Patenterteilung: **25.12.2019**
(21) Anmeldenummer: 15760100.6
(22) Anmeldetag: 18.08.2015
(51) Int. Cl.: B01J 19/00

(54) **VERFAHREN ZUR PHOSGENIERUNG VON HYDROXYL-, THIOL-, AMINO- UND/ODER FORMAMIDGRUPPEN ENTHALTENDEN VERBINDUNGEN**
METHOD FOR THE PHOSGENATION OF COMPOUNDS COMPRISING HYDROXYL, THIOL, AMINO AND/OR FORMAMIDE GROUPS
PROCÉDÉ DE PHOSGÉNATION DE COMPOSÉS CONTENANT DES GROUPES HYDROXYLE, THIOL, AMINO ET/OU FORMAMIDE

(30) Priorität: 20.08.2014 DE 102014111903
(43) Veröffentlichungstag der Anmeldung: 26.07.2017
(73) Patentinhaber: Bayer Aktiengesellschaft, 51373 Leverkusen (DE)
(72) Erfinder: ROGGAN, Jens Stefan, 50679 Köln (DE); MLECZKO, Leslaw, 41542 Dormagen (DE); METAXAS, Konstantinos, 50670 Köln (DE); GOTTFRIED, Michael, 42349 Wuppertal (DE); PECKERMANN, Ilja, 50735 Köln (DE); SCHUBERT, Stephan, 51375 Leverkusen (DE); BARTH, Ekkehard, 51061 Köln (DE)
(74) Vertreter: BIP Patents
(86) Internationale Anmeldenummer: PCT/EP2015/068883
(87) Internationale Veröffentlichungsnummer: WO 2016/026826

(56) Entgegenhaltungen:
- EP-A1- 0 520 238
- EP-A2- 1 112 997
- WO-A1-2010/076208
- US-A1- 2013 099 166

## Beschreibung

Die Arbeiten, die zu dieser Erfindung geführt haben, wurden gemäß der Finanzhilfevereinbarung Nr. 245988 im Zuge des Siebten Rahmenprogramms der Europäischen Union (FP7/2007-2013) gefördert.

Die vorliegende Erfindung betrifft ein Verfahren zur Reaktion einer ersten Verbindung mit einer zweiten Verbindung, wobei die erste Verbindung eine Gefahrstoffkennzeichnung gemäß GHS von GHS06 aufweist und aus der Reaktion von mindestens einer ersten und einer zweiten Vorläuferverbindung erhältlich ist und wobei die zweite Verbindung mit der ersten Verbindung zu einer chemischen Reaktion in der Lage ist. Insbesondere handelt es sich um Phosgenierungsverfahren von aromatischen Alkoholen. Sie betrifft weiterhin einen Reaktor, der zur Durchführung des erfindungsgemäßen Verfahrens geeignet ist.

Phosgen (COCl₂) ist ein Schlüsselreagenz in der Herstellung von Pharmazeutika, Polyurethanen und Polycarbonaten. Es ist eine sehr reaktive aber auch sehr toxische Chemikalie, und der großtechnische Herstellungsprozess birgt aufgrund der in einer Anlage vorhandenen Phosgenmengen (hold-up) immer Risiken für die Umwelt im Falle einer unbeabsichtigten Freisetzung durch Lecks in Rohrleitungen oder anderen Schäden an Anlagenteilen.

Ein Beispiel für die großtechnische Verwendung von Phosgen als Schlüsselreagenz ist die Herstellung von Diphenylcarbonat (DPC). DPC ist ein wichtiges Zwischenprodukt für die Synthese qualitativ hochwertiger Polycarbonate, zum Beispiel durch Umesterung mit Bisphenol A. Die Synthese von DPC ausgehend von Phenol und Phosgen (Direktphosgenierung) verläuft in zwei Schritten: der erste Schritt umfasst die Herstellung von Phosgen in einer Gasphasenreaktion aus Kohlenmonoxid und Chlor, welche typischerweise über Aktivkohlekatalysatoren in einem Multiröhren-Festbettreaktor erfolgt / durchgeführt wird. Je nach Siedetemperatur des Kühlmediums in den Reaktoren unterscheidet man zwischen einer Phosgenherstellung in Kaltvereinigern oder Heissvereinigern. Durch Reaktion von Phenol mit Phosgen in Gegenwart eines geeigneten Katalysators erhält man schließlich DPC. Die DPC-Herstellung über Phenol-Direktphosgenierung bietet dabei im Vergleich zu den herkömmlichen Phasengrenzflächenverfahren (Reaktion von Natriumphenolat mit Phosgen) den Vorteil, dass die Bildung großer Mengen an NaCl-Abfallprodukten vermieden wird.

Sowohl die Phosgen- als auch die DPC-Synthese sind stark exotherme Reaktionen mit Reaktionsenthalpien von -107 und -54 kJ/mol. Insbesondere die Exothermie der Phosgensynthese in der Gasphase erfordert effiziente Kühlungssysteme, doch lassen sich sogenannten Hot Spots im Reaktor mit lokalen Temperaturen von über 500 °C nicht verhindern (vgl. Mitchell et al., Catal. Sci. Technol., 2012). Das Auftreten von Temperaturen von über 300 °C führt nicht nur zu einer erhöhten Materialbeanspruchung im Reaktor, sondern beeinflusst in negativer Weise die Gleichgewichtsreaktion der Phosgenbildung (der Zerfall des Phosgens überwiegt bei über 300 °C) und erhöht zudem die Desaktivierungsrate des Katalysators, so dass insgesamt die Raum-Zeit-Ausbeute und Prozess-Effizient sinken.

EP0520238 offenbart ein Verfahren und einen Reaktor zur Reaktion von Phosgen (GHS06 Verbindung) mit einer Hydroxylgruppen enthaltenden Verbindung (Phenol).

WO 2003/072237 A1 offenbart einen Reaktor zur Herstellung von Phosgen durch Gasphasenreaktion von Kohlenmonoxid und Chlor in Gegenwart eines Feststoffkatalysators, mit einem Bündel von parallel zueinander, in Reaktorlängsrichtung angeordneten Kontaktrohren, die an ihren Enden in Rohrböden befestigt sind, mit je einer Haube an beiden Enden des Reaktors, sowie mit senkrecht zur Reaktorlängsrichtung im Zwischenraum zwischen den Kontaktrohren angeordneten Umlenkblechen, die alternierend einander gegenüberliegende Durchtrittsöffnungen an der Reaktorinnenwand freilassen, wobei die Kontaktrohre mit dem Feststoffkatalysator befüllt sind, das gasförmige Reaktionsgemisch von einem Reaktorende über eine Haube durch die Kontaktrohre geleitet und vom entgegengesetzten Reaktorende über die zweite Haube abgezogen und durch den Zwischenraum um die Kontaktrohre ein flüssiges Wärmetauschmittel geleitet wird, wobei der Reaktor im Bereich der Durchtrittsöffnungen unberohrt ist.

Auch das mit diesem Reaktor beschriebene Verfahren produziert große Mengen an Phosgen, die allerdings räumlich getrennt und zeitversetzt weiter umgesetzt werden.

US 2013/303783 A1 betrifft ein kontinuierliches Verfahren zur Herstellung von CO und Cl₂ und dem Verbrauch des so generierten Phosgens in einer Flüssigphasenreaktion, um organische Produkte P zu erhalten. Das Verfahren wird in zwei aufeinanderfolgenden Reaktoren R1 und R2 implementiert, wobei der erste Reaktor R1 ein Reaktor für die katalytische Synthese von Phosgen aus CO und Cl₂-Gas ist und der zweite Reaktor ein Kolbenreaktor mit einer mechanischen Rühreinrichtung ist.

Betrachtet man die aktuelle Entwicklung, so wird ein Bedarf an einem Verfahren mit reduziertem Phosgen-Holdup erkennbar. Im Rahmen der vorliegenden Erfindung wird ein solches Verfahren bereitgestellt. Insbesondere hat sich die Erfindung die Aufgabe gestellt, ein Phosgenierungsverfahren bereitzustellen, bei dem möglichst geringe Mengen an freiem Phosgen in der Reaktionsanlage vorliegen.

Erfindungsgemäß gelöst wird diese Aufgabe durch ein Verfahren zur Reaktion einer ersten Verbindung mit einer zweiten Verbindung,
wobei die erste Verbindung eine Gefahrstoffkennzeichnung gemäß GHS von GHS06 aufweist und aus der Reaktion von mindestens einer ersten fluiden Vorläuferverbindung und einer zweiten fluiden Vorläuferverbindung erhältlich ist
und wobei die zweite Verbindung mit der ersten Verbindung zu einer chemischen Reaktion in der Lage ist,
umfassend die Schritte:
(I) Bereitstellen einer flüssigen Phase, welche die zweite Verbindung enthält, in einem Reaktor mit einem in Schwerkraftrichtung gesehen oberen und unteren Ende;
(II) Bereitstellen eines Kontaktrohres mit einem oberen und unteren Ende in dem Reaktor, wobei
   das untere Ende des Kontaktrohres in die die zweite Verbindung enthaltende flüssige Phase eintaucht und
   in dem Kontaktrohr ein Katalysatorbett vorliegt, welches eingerichtet ist, um die Reaktion der ersten und zweiten Vorläuferverbindung zur ersten Verbindung zu katalysieren;
(III) Einleiten der ersten und zweiten Vorläuferverbindung durch das Kontaktrohr, wobei die im Kontaktrohr gebildete erste Verbindung aus dem unteren Ende des Kontaktrohres austritt und die zweite Verbindung enthaltende flüssige Phase kontaktiert.

Es ist erfindungsgemäß vorgesehen, dass die erste Verbindung eine Gefahrstoffkennzeichnung gemäß GHS (Globally Harmonized System of Classification, Labelling and Packaging of Chemicals der Vereinten Nationen) von GHS06 aufweist. In der Europäischen Union existiert hierzu das Regelwerk der Verordnung (EG) Nr. 1272/2008, auch CLP-Verordnung genannt. Die Einstufung GHS06 weist auf giftige oder sehr giftige Stoffe hin.

Hinsichtlich der ersten fluiden Vorläuferverbindung und der zweiten fluiden Vorläuferverbindung sind erfindungsgemäß Gase und Flüssigkeiten, einschließlich Lösungen von Feststoffen in einem Lösungsmittel, vorgesehen.

Insbesondere kann die erste Verbindung Phosgen sein, die erste fluide Vorläuferverbindung Kohlenmonoxid sein, die zweite fluide Vorläuferverbindung Chlor sein, der im Katalysatorbett vorliegende Katalysator ein Aktivkohle-Katalysator sein und die zweite Verbindung eine Hydroxyl-, Thiol-, Amino- und/oder Formamidgruppen enthaltende Verbindung sein.

Es ist ferner bevorzugt, dass der Aktivkohle-Katalysator eine BET-Oberfläche von ≥ 300 bis ≤ 2000 m²/g und einen d₉₀-Wert der Partikelgrößenverteilung von 25 µm bis 4 mm aufweist. Die BET-Oberfläche liegt mehr bevorzugt in einem Bereich von ≥ 800 bis ≤ 1200 m²/g; der d₉₀-Wert der Partikelgrößenverteilung in einem Bereich von ≥ 25 µm bis ≤ 4 mm. Im kleineren Reaktionsmaßstab ist insbesondere ein d₉₀-Wert der Partikelgrößenverteilung von ≥ 40 µm bis ≤ 120 µm günstig; im großtechnischen Maßstab sind es Aktivkohle-Extrudate mit einem d₉₀-Wert der Partikelgrößenverteilung von ≥1 mm bis ≤ 4 mm.

Aufgrund der herausragenden Bedeutung der Reaktion von Phosgen mit einer Hydroxyl-, Thiol-, Amino- und/oder Formamidgruppen enthaltenden Verbindung wird die vorliegende Erfindung im Zusammenhang mit dieser ersten und zweiten Verbindung erläutert, ohne jedoch hierauf beschränkt zu sein.

Im erfindungsgemäßen Verfahren tritt Phosgen nur als vergleichsweise kurzlebiges Intermediat auf. Das Gasgemisch aus Kohlenmonoxid und Chlor, reagiert bei Durchtritt durch ein Kontaktrohr zu Phosgen. Das *in situ* gebildete Phosgen tritt am unteren Ende des Kontaktrohres aus, steigt nach oben und reagiert mit der Hydroxyl-, Thiol-, Amino- und/oder Formamidgruppen enthaltenden Verbindung.

Durch das erfindungsgemäße Verfahren kann vermieden werden, dass in der Reaktionsanlage größere Mengen an Phosgen vorliegen. Die Hydroxyl-, Thiol-, Amino- und/oder Formamidgruppen enthaltende Verbindung dient ferner der Abfuhr der Reaktionswärme. Zusätzlich wird die Bildung von NaCl als Nebenprodukt gegenüber dem konventionellen Phasentransfer-Verfahren vermieden. Aus der entstehenden HCl kann über bekannte Recycling-Verfahren Chlor rückgewonnen werden. Insgesamt erhöht sich durch die Integration von zwei Reaktionen in einem Verfahren die Raum-Zeit-Ausbeute des Verfahrens über einen längeren Zeitraum und die thermische Belastung der Anlage wird verringert.

Gemäß Schritt (I) des erfindungsgemäßen Verfahrens wird eine flüssige Phase bereitgestellt. Diese kann insbesondere aufgeschmolzenes Edukt oder in einem Lösungsmittel gelöstes Edukt enthalten. Die Bauform des Reaktors ist zunächst nicht weiter festgelegt und kann beispielsweise ein Rohrreaktor für den kontinuierlichen Betrieb oder ein Kesselreaktor für eine diskontinuierliche Fahrweise sein. Der Reaktor weist ein oberes und ein unteres Ende auf, wobei die Schwerkraftrichtung als Bezug dient.

Schritt (II) des erfindungsgemäßen Verfahrens beinhaltet das Bereitstellen (mindestens) eines Kontaktrohres in dem Reaktor, wobei das Kontaktrohr ebenfalls in Bezug auf die Schwerkraftrichtung ein oberes und ein unteres Ende aufweist. Das untere Ende des Kontaktrohres taucht hierbei in die flüssige Phase ein. Zur Optimierung der Verfahrenseffizienz kann das Kontaktrohr möglichst weit in die flüssige Phase eintauchen.

Im Schritt (III) des erfindungsgemäßen Verfahrens werden Kohlenmonoxid und Chlor in das Kontaktrohr eingeleitet und reagieren dort zu Phosgen. Bedingt durch den Gasdruck der Eduktgase tritt das gasförmige Phosgen am unteren Ende des Kontaktrohrs aus, kontaktiert die flüssige Phase und steigt nach oben. Hierbei findet die Reaktion mit der Hydroxyl-, Thiol-, Amino- und/oder Formamidgruppen enthaltenden Verbindung statt.

Durch die Wahl geeigneter Flüssigkeits- und Gasdrücke in den beiden Reaktionsräumen kann das Übertreten von flüssigen Reaktanden in das Innere des Kontaktrohrs unterbunden werden.

Der Boden des Kontaktrohres kann beispielsweise durch eine Membran oder eine Fritte gebildet werden.

Beispiele für geeignete Hydroxyl-, Thiol-, Amino- und/oder Formamidgruppen enthaltenden Verbindungen sind aromatische Alkohole wie Phenol, aliphatische Alkohole, primäre aromatische Amine, sekundäre aromatische Amine, primäre aliphatische Amine, sekundäre aliphatische Amine, N,N-Dimethylformamid und N-Methylformanilid. Insbesondere aromatische und aliphatische Alkohole und Formamide sind bevorzugt; Erstere wegen der Anwendung der Reaktionsprodukte in der Polycarbonatherstellung und Letztere wegen ihres Einsatzes in Vilsmeyer-Haack-Formylierungen. Weiterhin bevorzugt sind primäre Amine, da sie durch Phosgenierung in die korrespondierenden Isocyanate überführt werden können, welche in der Polyurethanherstellung verwendet werden.

Insgesamt kann das Kontaktrohr auch als Festbettreaktor für eine Gasreaktion angesehen werden.

Gegen Korrosion empfindliche Oberflächen im Reaktor können zum Beispiel durch eine Edelstahl- oder SiO₂-Beschichtung geschützt werden.

Hinsichtlich der Reaktionsbedingungen im erfindungsgemäßen Verfahren kann die Reaktionstemperatur für die Phosgensynthese vorteilhafterweise zwischen 80 und 300 °C und für die Phosgenierung (insbesondere von Phenol) zwischen 80 und 300 °C liegen. Besonders bevorzugt ist eine Reaktionstemperatur in der flüssigen Phase im Reaktor von 190 bis 210 °C.

Damit die flüssige Phase besonders effektiv als Wärmeabfuhrmedium dienen kann, ist es günstig, wenn die Temperatur für die Phosgensynthese und die Temperatur in der flüssigen Phase um nicht mehr als 20 °C, 10 °C oder 5 °C voneinander abweichen. Es ist besonders günstig, wenn die Temperatur der Phosgensynthese (innerhalb der üblichen prozessbedingten Schwankungen) gleich der Temperatur in der flüssigen Phase ist.

Bevorzugt ist ein molarer Überschuss an Phenol von ≥ 4 bis ≤ 6.

Weitere Ausführungsformen und Aspekte der vorliegenden Erfindung werden nachfolgend erläutert. Sie können beliebig miteinander kombiniert werden, sofern sich aus dem Kontext nicht eindeutig das Gegenteil ergibt.

In einer Ausführungsform des erfindungsgemäßen Verfahrens wird das Verfahren in einem Reaktor durchgeführt wird, welcher umfasst:
eine Haube am oberen Ende des Reaktors, welche im Inneren des Reaktors durch einen Rohrboden begrenzt wird;
eine Mehrzahl von Kontaktrohren , welche in Längsrichtung des Reaktors angeordnet sind, wobei die Kontaktrohre mit ihren oberen Enden an dem Rohrboden befestigt sind;
und wobei die die zweite Verbindung enthaltende flüssige Phase in dem Zwischenraum um die Kontaktrohre bereitgestellt wird.

In einer weiteren Ausführungsform des erfindungsgemäßen Verfahrens liegt in der die zweite Verbindung enthaltenden flüssigen Phase weiterhin ein Katalysator vor. Der Katalysator, insbesondere für die Reaktion von Phosgen mit der Hydroxyl-, Thiol-, Amino- und/oder Formamidgruppen enthaltenden Verbindung, ist vorzugsweise in dem Reaktionsmedium, welches sich im zweiten Reaktionsraum befindet, gelöst. Im Fall der Phosgenierung von aromatischen Alkoholen wie Phenol können beispielsweise TiCl₄ oder Pyridin eingesetzt werden.

In einer weiteren Ausführungsform des erfindungsgemäßen Verfahrens ist die Hydroxyl-, Thiol-, Amino- und/oder Formamidgruppen enthaltenden Verbindung Phenol, Dimethylformamid oder N-Methylformanilid.

In einer weiteren Ausführungsform des erfindungsgemäßen Verfahrens weisen das Kontaktrohr oder die Kontaktrohre ein Verhältnis von Länge zu Durchmesser von ≥ 15:1 bis ≤ 1600:1 auf. Vorzugsweise liegt das Verhältnis in einem Bereich von ≥ 50:1 bis ≤ 350:1.

In einer weiteren Ausführungsform des erfindungsgemäßen Verfahrens beträgt die Summe der Partialdrücke der ersten Vorläuferverbindung und der zweiten Vorläuferverbindung ≥ 1 bar bis ≤ 26 bar. Bevorzugt ist eine Summe dieser Partialdrücke von ≥ 11 bis ≤ 15 bar. Unabhängig davon ist es bevorzugt, dass der Druck in dem Teil des Reaktors, der die die zweite Verbindung enthaltende flüssige Phase enthält, um ≥ 3 bis ≤ 6 bar, insbesondere ≥ 4 bis ≤ 5 bar, niedriger als die Summe der Partialdrücke der ersten Vorläuferverbindung und der zweiten Vorläuferverbindung ist.

Die Erfindung betrifft weiterhin einen Reaktor zur Reaktion von Phosgen mit Hydroxyl-, Thiol-, Amino- und/oder Formamidgruppen enthaltenden Verbindungen, umfassend:
eine Haube am oberen Ende des Reaktors, welche im Inneren des Reaktors durch einen Rohrboden begrenzt wird;
eine Mehrzahl von Kontaktrohren, welche in Längsrichtung des Reaktors angeordnet sind, wobei die Kontaktrohre mit ihren oberen Enden an dem Rohrboden befestigt sind,
wobei
in den Kontaktrohren ein Katalysatorbett vorliegt, welches eingerichtet ist, um die Reaktion von Kohlenmonoxid und Chlor zu katalysieren;
der Reaktor eingerichtet ist, um Kohlenmonoxid- und Chlorgas in den zwischen Haube und Rohrboden gebildeten Raum einzuleiten, so dass diese Gase durch die Kontaktrohre strömen;
der Reaktor eingerichtet ist, um eine Hydroxyl-, Thiol-, Amino- und/oder Formamidgruppen enthaltende Verbindung in den in Zwischenraum um die Kontaktrohre einzuleiten und flüssige Reaktionsprodukte dieser Verbindung mit Phosgen aus diesem Zwischenraum zu entnehmen und
der Reaktor weiterhin eingerichtet ist, um auf der der Haube abgewandten Seite des Rohrbodens gasförmige Reaktionsprodukte zu entnehmen.

In einer Ausführungsform des erfindungsgemäßen Reaktors weist der Aktivkohle-Katalysator eine BET-Oberfläche von ≥ 300 bis ≤ 2000 m²/g und einen d₉₀-Wert der Partikelgrößenverteilung von 25 µm bis 4 mm auf. Die BET-Oberfläche liegt mehr bevorzugt in einem Bereich von ≥ 800 bis ≤ 1200 m²/g, der d₉₀-Wert der Partikelgrößenverteilung in einem Bereich von ≥ 25 µm bis ≤ 4 mm. Im kleineren Reaktionsmaßstab ist insbesondere ein d₉₀-Wert der Partikelgrößenverteilung von ≥ 40 µm bis ≤ 120 µm günstig; im großtechnischen Maßstab sind es Aktivkohle-Extrudate mit einem d₉₀-Wert der Partikelgrößenverteilung von ≥ 3 mm bis ≤ 4 mm.

In einer weiteren Ausführungsform des erfindungsgemäßen Reaktors weisen die Kontaktrohre ein Verhältnis von Länge zu Durchmesser von ≥ 15:1 bis ≤ 1600:1 auf. Vorzugsweise liegt das Verhältnis in einem Bereich von ≥ 50:1 bis ≤ 350:1.

Die vorliegende Erfindung wird anhand der nachfolgenden Figuren und Beispiele näher erläutert, ohne jedoch darauf beschränkt zu sein. Es zeigen:
FIG. 1 einen Querschnitt durch einen Reaktor für das erfindungsgemäße Verfahren
FIG. 2 einen Querschnitt durch einen erfindungsgemäßen Reaktor

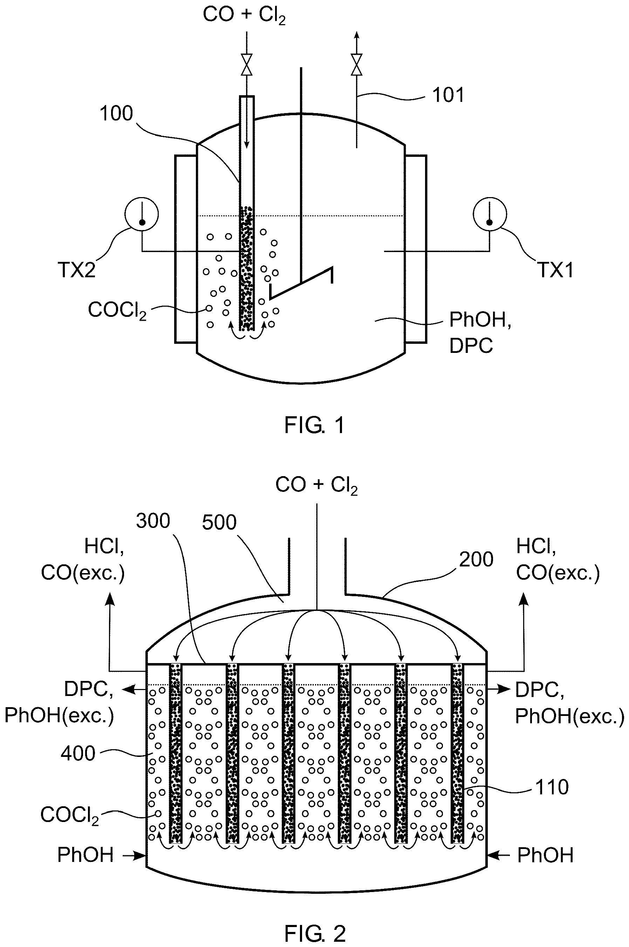

FIG. 1 zeigt einen schematischen Querschnitt durch einen Reaktor, in dem das erfindungsgemäße Verfahren durchgeführt wird. Ein Rührkesselreaktor mit seitlichem Heiz-/Kühlmantel wurde mit geschmolzenem Phenol beschickt. Die Höhe des Flüssigkeitsspiegels ist durch die gepunktete Linie im Reaktor dargestellt. Im Reaktor ist ein Kontaktrohr 100 mit Aktivkohle-Katalysatorfestbett angeordnet, wobei das untere Ende des Kontaktrohrs 100 in das geschmolzene Phenol eintaucht.

Über die Leitung 101 kann in den Reaktor eingebrachtes Gas wieder entfernt werden. Das Gas kann beispielsweise Stickstoff sein, wenn der Reaktor vor Beginn der Reaktion inertisiert wird. Weiterhin können Substanzen über die Leitung 101 in den Reaktor eingebracht werden. Hierbei kann es sich insbesondere um Katalysatoren für die Herstellung von DPC wie beispielsweise TiCl₄ handeln.

Der Temperaturfühler TX1 misst die Temperatur in der flüssigen Phase innerhalb des Reaktors. Die Temperatur im Inneren des Kontaktrohrs wird durch den Temperaturfühler TX2 gemessen.

Beim Durchleiten von Kohlenmonoxid und Chlor durch das Kontaktrohr 100 wird Phosgen gebildet, welches in Form von Gasblasen aus dem unteren Ende des Kontaktrohrs 100 austritt und nach oben durch das Phenol steigt. Hierbei wird DPC gebildet.

FIG. 2 zeigt einen schematischen Querschnitt durch einen erfindungsgemäßen Reaktor, in dem das erfindungsgemäße Verfahren durchgeführt wird. An seinem in Bezug auf die Schwerkraftrichtung gesehen oberen Ende befindet sich eine Haube 200. Im Reaktor liegt ferner ein Rohrboden 300 vor, welcher die Haube 200 im Inneren begrenzt. Dadurch wird ein Gasraum 500 gebildet.

Der Rohrboden 300 trägt mehrere Kontaktrohre 110, welche mit ihren oberen Enden am Rohrboden 300 befestigt sind. Die Kontaktrohre 110 sind in Längsrichtung des Reaktors angeordnet. Auf der Seite, welche dem Rohrboden 300 abgewandt ist, bilden die Kontaktrohre 110 einen Zwischenraum 400.

Der Zwischenraum 400 wird mit flüssigem Phenol beschickt. Die Höhe des Flüssigkeitsspiegels ist durch die gepunktete Linie im Reaktor dargestellt. Gleichzeitig werden Kohlenmonoxid und Chlorgas in die Haube 200 eingeleitet, vermischen sich im Raum 500 gelangen durch die Öffnungen am oberen Ende der Kontaktrohre 110 in diese hinein. Wie bereits zuvor geschildert wird in den Kontaktrohren 110 Phosgen gebildet, welches an den Öffnungen der unteren Enden der Kontaktrohre 110 austritt, durch das Phenol nach oben steigt und dabei mit dem Phenol unter Bildung von DPC reagiert.

Am oberen Ende des Flüssigkeitsspiegels werden DPC und überschüssiges Phenol ("PhOH(exc.)") entnommen. Oberhalb des Flüssigkeitsspiegels auf der der Haube 200 abgewandten Seite des Rohrbodens 300 werden die gasförmigen Komponenten HCl und überschüssiges Kohlenmonoxid ("CO(exc.)") entnommen.

Der Rohrboden 300 weist neben den Öffnungen, die durch die oberen Enden der Kontaktrohre 110 gebildet werden, keine weiteren Öffnungen auf. Auf diese Weise kann der Gasraum 500 vom Zwischenraum 400 getrennt werden. Dieses hat den Vorteil, dass der Kontakt von Chlorgas mit Phenol und die Bildung von Chloraromaten als Nebenprodukte vermieden werden kann.

### Beispiele

### Durchführen des erfindungsgemäßen Verfahrens im Labormaßstab

Der Versuchsaufbau erfolgte analog zu der in FIG. 1 gezeigten schematischen Anordnung. Ein 600 mL Druckbehälter, ausgestattet mit einem Begasungsrührer und Innenthermometer (vgl. TX1 in FIG. 1), wurde mit 280.4 g (2.98 mol) Phenol beladen und Letzteres durch Erhitzen auf ca. 45-50 °C aufgeschmolzen. Die Katalysatorkartusche (vgl. 100 in FIG. 1; Länge: 118 mm, Durchmesser: 12 mm) wurde mit 2.59 g Aktivkohlepulver (Partikelgröße 45 - 125 µm) beschickt und an den Deckel des Druckbehälters gasdicht festgeschraubt. Der Boden der Kartusche war mit einer Bohrung (0.5 mm Durchmesser) versehen. In einem Stickstoff-Strom wurde der mit der integrierten Katalysatorkartusche (vgl. 100 in FIG. 1) versehene Reaktordeckel aufgeschraubt und somit die Katalysatorkartusche in die Phenolschmelze eingetaucht. Die Messung der Innentemperatur der Kartusche erfolgte über ein weiteres Thermoelement (vgl. TX2 in FIG. 2) in mittlerer Kartuschenposition. Der Reaktorausgang (vgl. Leitung 101 in FIG. 1) war zu diesem Zeitpunkt noch geöffnet und der Reaktor wurde durch den steten N₂-Strom (6.0 mL/min) 30 min lang unter Rühren inertisiert. Anschließend wurden über das in dieser Leitung vorhandene Ventil (vgl. Leitung 101 in FIG. 1) 0.1 mL TiCl₄ zum flüssigen PhOH zugegeben. Es wurde nun unter stetem N₂-Fluss (6.0 mL/min) auf 200 °C erhitzt. Das Reaktor-Auslassventil (vgl. Leitung 101 in FIG. 1) wurde hierbei bei einer Innentemperatur von TX1 = 130 °C verschlossen. Nach Erreichen von 200 °C Reaktor-Innentemperatur wurden 4.5 mL/min CO und 4.3 mL/min Cl₂ unter gleichzeitigem Abbruch der N₂-Zugabe zudosiert (Katalysatorkartusche; vgl. 100 in FIG. 1). Nach Einleitung von insgesamt 1.12 L CO (0,050 mol) und 1.06 L Cl₂ (0.047 mol) wurde die Cl₂ und CO Zugabe beendet und auf 3.0 mL/min N₂ umgestellt. Der Druck im Reaktor betrug zu diesem Zeitpunkt 12 bar. Das Reaktionsgemisch wurde 60 min bei 200°C gerührt und dann über mehrere Stunden auf 50 °C abgekühlt. Nach Erreichen von 120 °C wurde der Reaktor entspannt. Unter weiterhin stetem N₂-Strom (3.0 mL/min) und Rühren (2000 Umin⁻¹) wurde der Reaktor über Nacht inertisiert und von möglichen Resten an HCl, COCl₂, CO oder Cl₂ befreit. Die Bildung von DPC wurde durch Analyse des erhaltenen Produktgemisches mittels Gaschromatographie nachgewiesen.

## Patentansprüche

1. Verfahren zur Reaktion einer ersten Verbindung mit einer zweiten Verbindung,
wobei die erste Verbindung eine Gefahrstoffkennzeichnung gemäß GHS von GHS06 aufweist und aus der Reaktion von mindestens einer ersten fluiden Vorläuferverbindung und einer zweiten fluiden Vorläuferverbindung erhältlich ist
und wobei die zweite Verbindung mit der ersten Verbindung zu einer chemischen Reaktion in der Lage ist,
umfassend die Schritte:
(I) Bereitstellen einer flüssigen Phase, welche die zweite Verbindung enthält, in einem Reaktor mit einem in Schwerkraftrichtung gesehen oberen und unteren Ende;
(II) Bereitstellen eines Kontaktrohres (100, 110) mit einem oberen und unteren Ende in dem Reaktor, wobei
das untere Ende des Kontaktrohres in die die zweite Verbindung enthaltende flüssige Phase eintaucht und
in dem Kontaktrohr ein Katalysatorbett vorliegt, welches eingerichtet ist, um die Reaktion der ersten und zweiten Vorläuferverbindung zur ersten Verbindung zu katalysieren;
(III) Einleiten der ersten und zweiten Vorläuferverbindung durch das Kontaktrohr (100, 110), wobei die im Kontaktrohr gebildete erste Verbindung aus dem unteren Ende des Kontaktrohres austritt und die zweite Verbindung enthaltende flüssige Phase kontaktiert.

2. Verfahren gemäß Anspruch 1, wobei die erste Verbindung Phosgen ist, die erste Vorläuferverbindung Kohlenmonoxid ist, die zweite Vorläuferverbindung Chlor ist, der im Katalysatorbett vorliegende Katalysator ein Aktivkohle-Katalysator ist und die zweite Verbindung eine Hydroxyl-, Thiol-, Amino- und/oder Formamidgruppen enthaltende Verbindung ist.

3. Verfahren gemäß Anspruch 2, wobei der Aktivkohle-Katalysator eine BET-Oberfläche von ≥ 300 bis ≤ 2000 m²/g und einen d₉₀-wert der Partikelgrößenverteilung von 25µm bis 4mm aufweist.

4. Verfahren gemäß einem der Ansprüche 1 bis 3, wobei das Verfahren in einem Reaktor durchgeführt wird, welcher umfasst:
eine Haube (200) am oberen Ende des Reaktors, welche im Inneren des Reaktors durch einen Rohrboden (300) begrenzt wird;
eine Mehrzahl von Kontaktrohren (100, 110), welche in Längsrichtung des Reaktors angeordnet sind, wobei die Kontaktrohre mit ihren oberen Enden an dem Rohrboden (300) befestigt sind;
und wobei die die zweite Verbindung enthaltende flüssige Phase in dem Zwischenraum (400) um die Kontaktrohre (100, 110) bereitgestellt wird.

5. Verfahren gemäß einem der Ansprüche 1 bis 4, wobei in der die zweite Verbindung enthaltenden flüssigen Phase weiterhin ein Katalysator vorliegt.

6. Verfahren gemäß einem der Ansprüche 1 bis 5, wobei das Kontaktrohr oder die Kontaktrohre (100, 110) ein Verhältnis von Länge zu Durchmesser von ≥ 15:1 bis ≤ 1600:1 aufweisen.

7. Verfahren gemäß einem der Ansprüche 1 bis 5, wobei die Summe der Partialdrücke der ersten Vorläuferverbindung und der zweiten Vorläuferverbindung ≥ 1 bar bis ≤ 26 bar beträgt.

8. Reaktor zur Reaktion von Phosgen mit Hydroxyl-, Thiol-, Amino- und/oder Formamidgruppen enthaltenden Verbindungen, umfassend:
eine Haube (200) am oberen Ende des Reaktors, welche im Inneren des Reaktors durch einen Rohrboden (300) begrenzt wird;
eine Mehrzahl von Kontaktrohren (100, 110), welche in Längsrichtung des Reaktors angeordnet sind, wobei die Kontaktrohre mit ihren oberen Enden an dem Rohrboden (300) befestigt sind,
**dadurch gekennzeichnet, dass**
in den Kontaktrohren (100, 110) ein Aktivkohle-Katalysator vorliegt, welcher eingerichtet ist, um die Reaktion von Kohlenmonoxid und Chlor zu katalysieren;
der Reaktor eingerichtet ist, um Kohlenmonoxid- und Chlorgas in den zwischen Haube und Rohrboden gebildeten Raum (500) einzuleiten, so dass diese Gase durch die Kontaktrohre (100, 110) strömen;
der Reaktor eingerichtet ist, um eine Hydroxyl-, Thiol-, Amino- und/oder Formamidgruppen enthaltende Verbindung in den in Zwischenraum (400) um die Kontaktrohre (100, 110) einzuleiten und flüssige Reaktionsprodukte dieser Verbindung mit Phosgen aus diesem Zwischenraum zu entnehmen und
der Reaktor weiterhin eingerichtet ist, um auf der der Haube (200) abgewandten Seite des Rohrbodens (300) gasförmige Reaktionsprodukte zu entnehmen.

9. Reaktor gemäß Anspruch 8, wobei der Aktivkohle-Katalysator eine BET-Oberfläche von ≥ 300 bis ≤ 2000 m²/g und einen d₉₀-Wert der Partikelgrößenverteilung von 25µm bis 4mm aufweist.

10. Reaktor gemäß Anspruch 8 oder 9, wobei die Kontaktrohre (100, 110) ein Verhältnis von Länge zu Durchmesser von ≥ 15:1 bis ≤ 1600:1 aufweisen.

11. Verwendung eines Reaktors nach den Ansprüchen 8 bis 10 zur Reaktion von Phosgen mit Hydroxyl-, Thiol-, Amino- und/oder Formamidgruppen enthaltenden Verbindungen.

## Claims

1. Method of reacting a first compound with a second compound,
wherein the first compound has a GHS hazard identification of GHS06 and is obtainable from the reaction of at least one first fluid precursor compound and one second fluid precursor compound
and wherein the second compound is capable of a chemical reaction with the first compound,
comprising the steps of:
(I) providing a liquid phase containing the second compound in a reactor having an upper end and a lower end viewed in the direction of gravity;
(II) providing a contact tube (100, 110) having an upper end and a lower end in the reactor, wherein
the lower end of the contact tube is immersed into the liquid phase containing the second compound and
a catalyst bed present in the contact tube is set up to catalyze the reaction between the first and second precursor compounds to give the first compound;
(III) introducing the first and second precursor compounds through the contact tube (100, 110), with the first compound formed in the contact tube exiting from the lower end of the contact tube and coming into contact with liquid phase containing the second compound.

2. Method according to Claim 1, wherein the first compound is phosgene, the first precursor compound is carbon monoxide, the second precursor compound is chlorine, the catalyst present in the catalyst bed is an activated carbon catalyst and the second compound is a compound containing hydroxyl, thiol, amino and/or formamide groups.

3. Method according to Claim 2, wherein the activated carbon catalyst has a BET surface area of ≥ 300 to ≤ 2000 m²/g and a d₉₀ of the particle size distribution of 25 µm to 4 mm.

4. Method according to any of Claims 1 to 3, wherein the method is conducted in a reactor comprising:
a hood (200) at the upper end of the reactor, bounded by a tube plate (300) within the reactor;
a multitude of contact tubes (100, 110) arranged in longitudinal direction of the reactor, the contact tubes being secured on the tube plate (300) by their upper ends;
and wherein the liquid phase containing the second compound is provided in the interspace (400) around the contact tubes (100, 110).

5. Method according to any of Claims 1 to 4, wherein a catalyst is additionally present in the liquid phase containing the second compound.

6. Method according to any of Claims 1 to 5, wherein the contact tube(s) (100, 110) has/have a ratio of length to diameter of ≥ 15:1 to ≤ 1600:1.

7. Method according to any of Claims 1 to 5, wherein the sum total of the partial pressures of the first precursor compound and the second precursor compound is ≥ 1 bar to ≤ 26 bar.

8. Reactor for reaction of phosgene with compounds containing hydroxyl, thiol, amino and/or formamide groups, comprising:
a hood (200) at the upper end of the reactor, bounded by a tube plate (300) within the reactor;
a multitude of contact tubes (100, 110) arranged in longitudinal direction of the reactor, the contact tubes being secured on the tube plate (300) by their upper ends,
**characterized in that**
an activated carbon catalyst present in the contact tubes (100, 110) is set up to catalyze the reaction of carbon monoxide and chlorine;
the reactor is set up to introduce carbon monoxide and chlorine gas into the space (500) formed between the hood and tube plate, such that these gases flow through the contact tubes (100, 110);
the reactor is set up to introduce a compound containing hydroxyl, thiol, amino and/or formamide groups into the in interspace (400) around the contact tubes (100, 110) and to withdraw liquid reaction products of this compound with phosgene from this interspace and
the reactor is additionally set up to withdraw gaseous reaction products on the side of the tube plate (300) facing away from the hood (200).

9. Reactor according to Claim 8, wherein the activated carbon catalyst has a BET surface area of ≥ 300 to ≤ 2000 m²/g and a d₉₀ of the particle size distribution of 25 µm to 4 mm.

10. Reactor according to Claim 8 or 9, wherein the contact tubes (100, 110) have a ratio of length to diameter of ≥ 15:1 to ≤ 1600:1.

11. Use of a reactor according to Claims 8 to 10 for reaction of phosgene with compounds containing hydroxyl, thiol, amino and/or formamide groups.

## Revendications

1. Procédé de mise en réaction d'un premier composé avec un deuxième composé,
dans lequel le premier composé présente une désignation de substance dangereuse selon le SGH de SGH06, et peut être obtenu par la mise en réaction d'au moins un premier composé précurseur fluide et d'un deuxième composé précurseur fluide,
et dans lequel le deuxième composé est en mesure de réaliser une réaction chimique avec le premier composé, comprenant les étapes suivantes :
(I) la mise à disposition d'une phase liquide, qui contient le deuxième composé, dans un réacteur comprenant une extrémité supérieure et une extrémité inférieure dans la direction de la force de pesanteur ;
(II) la mise à disposition d'un tube de contact (100, 110) comprenant une extrémité supérieure et une extrémité inférieure dans le réacteur,
l'extrémité inférieure du tube de contact plongeant dans la phase liquide contenant le deuxième composé, et un lit catalytique étant présent dans le tube de contact, lequel est conçu pour catalyser la réaction du premier et du deuxième composé précurseur pour former le premier composé ;
(III) l'acheminement du premier et du deuxième composé précurseur au travers du tube de contact (100, 110), le premier composé formé dans le tube de contact sortant par l'extrémité inférieure du tube de contact, et rentrant en contact avec la phase liquide contenant le deuxième composé.

2. Procédé selon la revendication 1, dans lequel le premier composé est le phosgène, le premier composé précurseur est le monoxyde de carbone, le deuxième composé précurseur est le chlore, le catalyseur présent dans le lit catalytique est un catalyseur à base de charbon actif, et le deuxième composé est un composé contenant des groupes hydroxyle, thiol, amino et/ou formamide.

3. Procédé selon la revendication 2, dans lequel le catalyseur à base de charbon actif présente une surface BET de ≥ 300 à ≤ 2 000 m²/g et une valeur d₉₀ de la distribution des tailles de particules de 25 µm à 4 mm.

4. Procédé selon l'une quelconque des revendications 1 à 3, dans lequel le procédé est réalisé dans un réacteur qui comprend :
un collecteur (200) à l'extrémité supérieure du réacteur, qui est délimité par un plateau porte-tubes (300) à l'intérieur du réacteur ;
une pluralité de tubes de contact (100, 110), qui sont agencés dans la direction longitudinale du réacteur, les tubes de contact étant fixés avec leurs extrémités supérieures au niveau du plateau porte-tubes (300) ;
et la phase liquide contenant le deuxième composé étant mise à disposition dans l'espace intermédiaire (400) autour des tubes de contact (100, 110).

5. Procédé selon l'une quelconque des revendications 1 à 4, dans lequel un catalyseur est en outre présent dans la phase liquide contenant le deuxième composé.

6. Procédé selon l'une quelconque des revendications 1 à 5, dans lequel le tube de contact ou les tubes de contact (100, 110) présentent un rapport longueur sur diamètre de ≥ 15:1 à ≤ 1 600:1.

7. Procédé selon l'une quelconque des revendications 1 à 5, dans lequel la somme des pressions partielles du premier composé précurseur et du deuxième composé précurseur est de ≥ 1 bar à ≤ 26 bar.

8. Réacteur pour la mise en réaction de phosgène avec des composés contenant des groupes hydroxyle, thiol, amino et/ou formamide, comprenant :
un collecteur (200) à l'extrémité supérieure du réacteur, qui est délimité par un plateau porte-tubes (300) à l'intérieur du réacteur ;
une pluralité de tubes de contact (100, 110), qui sont agencés dans la direction longitudinale du réacteur, les tubes de contact étant fixés avec leurs extrémités supérieures au niveau du plateau porte-tubes (300) ;
**caractérisé en ce que**
un catalyseur à base de charbon actif est présent dans les tubes de contact (100, 110), qui est conçu pour catalyser la réaction de monoxyde de carbone et de chlore ;
le réacteur est conçu pour acheminer du monoxyde de carbone et du chlore gazeux dans l'espace (500) formé entre le collecteur et le plateau porte-tubes, de telle sorte que ces gaz s'écoulent à travers les tubes de contact (100, 110) ;
le réacteur est conçu pour acheminer un composé contenant des groupes hydroxyle, thiol, amino et/ou formamide dans le dans l'espace intermédiaire (400) autour des tubes de contact (100, 110) et extraire des produits de réaction liquides de ce composé avec du phosgène à partir de cet espace intermédiaire, et
le réacteur est en outre conçu pour extraire des produits de réaction gazeux du côté opposé au collecteur (200) du plateau porte-tubes (300).

9. Réacteur selon la revendication 8, dans lequel le catalyseur à base de charbon actif présente une surface BET de ≥ 300 à ≤ 2 000 m²/g, et une valeur d₉₀ de la distribution des tailles de particules de 25 µm à 4 mm.

10. Réacteur selon la revendication 8 ou 9, dans lequel les tubes de contact (100, 110) présentent un rapport entre la longueur et le diamètre de ≥ 15:1 à ≤ 1 600:1.

11. Utilisation d'un réacteur selon les revendications 8 à 10 pour la mise en réaction de phosgène avec des composés contenant des groupes hydroxyle, thiol, amino et/ou formamide.
